(19) Europäisches Patentamt
European Patent Office
Office européen des brevets

(11) EP 4 769 628 A2

(12) EUROPEAN PATENT APPLICATION

(43) Date of publication:
**01.07.2026 Bulletin 2026/27**

(21) Application number: **25227153.1**

(22) Date of filing: **09.05.2022**

(51) International Patent Classification (IPC):
***H01M 10/0569*** *(2010.01)*

(52) Cooperative Patent Classification (CPC):
**H01M 10/0567; H01M 10/0525; H01M 10/0568; H01M 10/0569;** Y02E 60/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(62) Document number(s) of the earlier application(s) in accordance with Art. 76 EPC:
**22941020.4 / 4 354 577**

(71) Applicant: **Contemporary Amperex Technology (Hong Kong) Limited**
**Central, Central And Western District (HK)**

(72) Inventors:
- **PENG, Chang**
  **Ningde, 352100 (CN)**
- **CHEN, Peipei**
  **Ningde, 352100 (CN)**
- **WU, Degui**
  **Ningde, 352100 (CN)**
- **ZOU, Hailin**
  **Ningde, 352100 (CN)**

(74) Representative: **Gong, Jinping**
**CocreateIP**
**Neumarkter Straße 21**
**81673 München (DE)**

Remarks:
This application was filed on 24.12.2025 as a divisional application to the application mentioned under INID code 62.

# (54) ELECTROLYTE, SECONDARY BATTERY, BATTERY MODULE, BATTERY PACK, AND ELECTRIC APPARATUS

(57) This application provides an electrolyte for lithium-ion secondary battery and a battery. The electrolyte includes a solvent, an additive composition, and a lithium salt. The additive composition includes a compound of formula I and at least one of compounds of formula II and formula III. The resulting battery has a high voltage and exhibits good cycling performance and storage performance at high temperature.

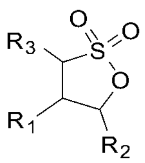
formula II

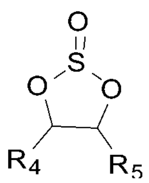
formula III

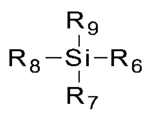
formula I

In the formulas, the symbols are as defined in the specification.

EP 4 769 628 A2

## Description

## TECHNICAL FIELD

This application relates to the field of lithium battery technologies, and in particular, to an electrolyte, a secondary battery, a battery module, a battery pack, and an electric apparatus.

## BACKGROUND

Featuring high operating potential, long service life, and environmental friendliness, lithium-ion batteries have emerged as the most popular energy storage system, widely used in battery electric vehicles, hybrid electric vehicles, smart power grids, and other fields. As the demand for endurance grows and people seek to alleviate their anxiety about the mileage of electric vehicles, there is an urgent need to develop a lithium-ion battery system with higher energy density.

In order to improve the energy density of batteries, increasing the operating potential of positive electrode materials has become a primary strategy for all researchers. However, if the operating voltage of the conventional ternary positive-electrode material is increased to more than 4.4 V, the electrolyte becomes susceptible to oxidation, resulting in significantly poor cell life performance. In the prior art, film-forming additives (for example, tetravinylsilicon) are typically added to the electrolyte to enhance the film formation on the negative electrode and reduce the damage to the negative electrode caused by the by-products resulting from the oxidation of the positive electrode. However, these additives lead to high film-forming impedance, which seriously degrades the initial impedance of the battery. Therefore, there is still a need to improve the existing electrolyte additives.

## SUMMARY

This application is made in view of the foregoing issue and intended to provide an electrolyte for lithium-ion secondary battery and a battery. The resulting battery has a high voltage and exhibits excellent cycling performance and storage performance at high temperature.

To achieve the foregoing objective, a first aspect of this application provides an electrolyte for lithium-ion secondary battery. The electrolyte includes a solvent, an additive composition, and a lithium salt and is characterized in that the additive composition includes a compound of formula I and at least one of compounds of formula II and formula III.

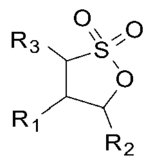

formula II

where $R_1$, $R_2$, and $R_3$ are each independently H or F, and at least one of $R_1$, $R_2$, and $R_3$ is F;

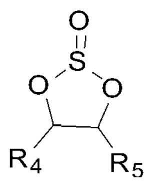

formula III

where $R_4$ and $R_5$ are each independently hydrogen, vinyl, allyl, oxyvinyl, or oxyallyl, and at least one of $R_4$ and $R_5$ is not H; and

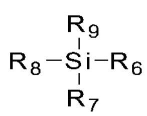

formula I

where $R_6$, $R_7$, $R_8$, and $R_9$ are each independently selected from one or more of allyl, vinyl, or allyl butyl.

Therefore, the electrolyte of this application, by including the compound I and compound II or III, ensures that the resulting battery has a high voltage and exhibits good cycling performance and storage performance at high temperature.

In any embodiment, the compound of formula I is:

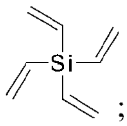 ;

the compound of formula II is:

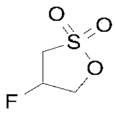 , 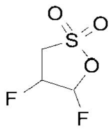 , or 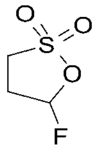 ;

and

the compound of formula III is:

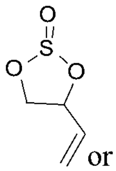 or 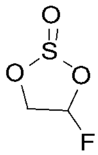 .

Therefore, the electrolyte of this application, by including the compound I and compound II or III of the foregoing structure, further enables the corresponding battery to have a high voltage and exhibit good cycling performance and storage performance at high temperature.

In any embodiment, the percentage of the compound of formula I in the electrolyte is a wt%, and the percentage of the compound of formula II, the compound of formula III, or the mixture of the two, in the electrolyte is b wt%, where

$$0.05 \leq a/b \leq 4.$$

Therefore, including the compound I and compound II or III with their respective percentages further enables the corresponding battery to have a high voltage and exhibit good cycling performance and storage performance at high temperature.

In any embodiment, the percentage of the compound of formula I in the electrolyte is a wt%, and the percentage of the compound of formula II, the compound of formula III, or the mixture of the two, in the electrolyte is b wt%, where

$$0.1 \leq a+b \leq 10.$$

Therefore, including the compound I and compound II or III with their respective percentages further enables the corresponding battery to have a high voltage and exhibit good cycling performance and storage performance at high temperature.

In any embodiment, the percentage of the compound of formula II, the compound of formula III, or a mixture of the two, in the electrolyte is 0.06wt% to 8wt%.

Therefore, including the compound II or III with the percentage further enables the corresponding battery to have a high voltage and exhibit good cycling performance and storage performance at high temperature.

In any embodiment, the solvent is a non-protonic organic solvent, the non-protonic organic solvent being selected from one or more of fluorinated and non-fluorinated cyclic and chain organic carbonate, fluorinated and non-fluorinated ether, fluorinated and non-fluorinated cyclic ether, fluorinated and non-fluorinated carboxylic acid ester, and fluorinated and non-fluorinated chain sulfone or cyclic sulfone compounds.

Therefore, including the solvent of this type further enables the corresponding battery to have a high voltage and

exhibit good cycling performance and storage performance at high temperature.

In any embodiment, the lithium salt is selected from one or more of lithium hexafluorophosphate ($LiPF_6$), lithium tetrafluoroborate ($LiBF_4$), lithium perchlorate ($LiClO_4$), lithium hexafluoroarsenate ($LiAsF_6$), lithium bis(fluorosulfonyl) imide (LiFSI), lithium bistrifluoromethanesulfonimide (LiTFSI), lithium trifluoromethanesulfonate (LiTFS), lithium difluoro(oxalato)borate (LiDFOB), lithium dioxalate borate (LiBOB), lithium difluorophosphate ($LiPO_2F_2$), lithium difluoro(dioxalato)phosphate (LiDFOP), and lithium tetrafluoro oxalato phosphate (LiTFOP), and is optionally one or two of lithium hexafluorophosphate and lithium bis(fluorosulfonyl)imide.

Therefore, including the lithium salt of this type further enables the corresponding battery to have a high voltage and exhibit good cycling performance and storage performance at high temperature.

In any embodiment, in the electrolyte:

the solvent is 60wt% to 85wt%.
the additive composition is 0.01wt% to 20wt%, and
the lithium salt is 10wt% to 40wt%; and
a total weight of the foregoing components is 100wt% and each wt% is based on a total weight of the electrolyte.

Therefore, including the components with their respective percentages further enables the corresponding battery to have a high voltage and exhibit good cycling performance and storage performance at high temperature.

A second aspect of this application further provides a secondary battery characterized by including: the electrolyte according to the first aspect of this application.

Therefore, the battery has a high voltage and exhibits good cycling performance and storage performance at high temperature.

A third aspect of this application provides a battery module, including the secondary battery in the second aspect of this application.

A fourth aspect of this application provides a battery pack including the battery module in the third aspect of this application.

A fifth aspect of this application provides an electric apparatus, including at least one selected from the secondary battery according to the second aspect of this application, the battery module according to the third aspect of this application, or the battery pack according to the fourth aspect of this application.

In this application, the electrolyte, by including the compound I and compound II or III, further ensures that the resulting battery has a high voltage and exhibits good cycling performance and storage performance at high temperature.

## BRIEF DESCRIPTION OF DRAWINGS

FIG. 1 is a schematic diagram of a secondary battery according to an embodiment of this application.
FIG. 2 is an exploded view of the secondary battery according to the embodiment of this application in FIG. 1.
FIG. 3 is a schematic diagram of a battery module according to an embodiment of this application.
FIG. 4 is a schematic diagram of a battery pack according to an embodiment of this application.
FIG. 5 is an exploded view of the battery pack according to the embodiment of this application in FIG. 4.
FIG. 6 is a schematic diagram of an electric apparatus with a secondary battery as a power source according to an embodiment of this application.

Reference numerals in the accompanying drawings are described as follows:
1. battery pack; 2. upper box body; 3. lower box body; 4. battery module; 5. secondary battery; 51. housing; 52. electrode assembly; and 53. top cover assembly.

## DESCRIPTION OF EMBODIMENTS

The following specifically discloses in detail embodiments of an electrolyte, a secondary battery, a battery module, a battery pack, and an electric apparatus of this application with appropriate reference to the accompanying drawings. However, there may be cases where unnecessary detailed descriptions are omitted. For example, detailed description of a well-known matter or repeated description of an actual identical structure has been omitted. This is to avoid unnecessarily prolonging the following description, for ease of understanding by persons skilled in the art. In addition, the accompanying drawings and the following descriptions are provided for persons skilled in the art to fully understand this application and are not intended to limit the subject matter recorded in the claims.

"Ranges" disclosed in this application are defined in the form of lower and upper limits. A given range is defined by

one lower limit and one upper limit selected, where the selected lower and upper limits define boundaries of that particular range. Ranges defined in this method may or may not include end values, and any combinations may be used, meaning any lower limit may be combined with any upper limit to form a range. For example, if ranges of 60-120 and 80-110 are provided for a specific parameter, it is understood that ranges of 60-110 and 80-120 can also be envisioned. In addition, if minimum values of a range are provided as 1 and 2, and maximum values of the range are provided as 3, 4, and 5, the following ranges can all be envisioned: 1-3, 1-4, 1-5, 2-3, 2-4, and 2-5. In this application, unless otherwise stated, a value range of "a-b" is a short representation of any combination of real numbers between a and b, where both a and b are real numbers. For example, a value range of "0-5" means that all real numbers in the range of "0-5" are listed herein, and "0-5" is just a short representation of a combination of these values. In addition, a parameter expressed as an integer greater than or equal to 2 is equivalent to disclosure that the parameter is, for example, an integer among 2, 3, 4, 5, 6, 7, 8, 9, 10, 11, 12, and so on.

Unless otherwise specified, all the embodiments and optional embodiments of this application can be combined with each other to form new technical solutions.

Unless otherwise specified, all the technical features and optional technical features of this application can be combined with each other to form new technical solutions.

Unless otherwise specified, all the steps in this application can be performed sequentially or randomly, and preferably, are performed sequentially. For example, a method including steps (a) and (b) indicates that the method may include steps (a) and (b) performed in order or may include steps (b) and (a) performed in order. For example, the foregoing method may further include step (c), which indicates that step (c) may be added to the method in any ordinal position, for example, the method may include steps (a), (b), and (c), steps (a), (c), and (b), steps (c), (a), and (b), or the like.

Unless otherwise specified, "include" and "contain" mentioned in this application are inclusive or may be exclusive. For example, the terms "include" and "contain" can mean that other unlisted components may also be included or contained, or only listed components are included or contained.

Unless otherwise specified, in this application, the term "or" is inclusive. For example, the phrase "A or B" means "A, B, or both A and B". More specifically, any one of the following conditions satisfies the condition "A or B": A is true (or present) and B is false (or not present); A is false (or not present) and B is true (or present); or both A and B are true (or present).

In order to improve the energy density of batteries, increasing the operating potential of positive electrode materials has become a primary strategy for all researchers. However, if the operating voltage of the conventional ternary positive-electrode material is increased to more than 4.4 V, the electrolyte becomes susceptible to oxidation, resulting in significantly poor cell life performance. In the prior art, film-forming additives (for example, tetravinylsilicon) are typically added to the electrolyte to enhance the film formation on the negative electrode and reduce the damage to the negative electrode caused by the by-products resulting from the oxidation of the positive electrode. However, these additives lead to high film-forming impedance, which seriously degrades the initial impedance of the battery. Therefore, there is still a need to improve the existing electrolyte additives. This application is made in view of the foregoing issue and intended to provide an electrolyte for lithium-ion secondary battery and a battery. The resulting battery has a high voltage and exhibits excellent cycling performance and storage performance at high temperature.

**Electrolyte for lithium-ion secondary battery**

In an embodiment of this application, this application provides an electrolyte for lithium-ion secondary battery. The electrolyte includes a solvent, an additive composition, and a lithium salt, and is characterized in that the additive composition includes a compound of formula I and at least one of compounds of formula II and formula III:

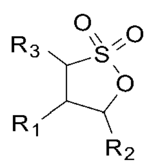

formula II

where $R_1$, $R_2$, and $R_3$ are each independently H or F, and at least one of $R_1$, $R_2$, and $R_3$ is F;

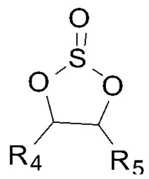

formula III

where $R_4$ and $R_5$ are each independently hydrogen, vinyl, allyl, oxyvinyl, or oxyallyl, and at least one of $R_4$ and $R_5$ is not H; and

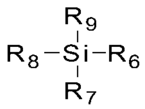

formula I

where $R_6$, $R_7$, $R_8$, and $R_9$ are each independently selected from one or more of allyl, vinyl, or allyl butyl.

Therefore, the electrolyte of this application, by including the compound I and compound II or III, allows the compound II or compound III to be preferably reduced to radicals containing sulfonic acid groups in the corresponding battery during film formation on the negative electrode and then to react with the compound I so as to form polypropylene sulfonate-polypropylene copolymer or polybutylene sulfonate-polypropylene copolymer with a proper molecular weight. In addition, the electrolyte further includes inorganic components such as silicate, sulfate, or lithium salt. The components forming the SEI membrane not only possess high ion conductivity but also exhibit moderate stiffness and flexibility. This can effectively protect the negative electrode interface, thus resisting the damage from the by-product generated by subsequent positive electrode oxidation to the negative electrode SEI membrane, and ensuring that the resulting battery has a high voltage and exhibits good cycling performance and storage performance at high temperature.

In some embodiments, the compound of formula I is:

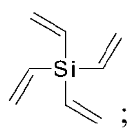 ;

the compound of formula II is:

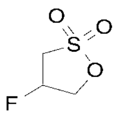 , 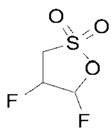 , or 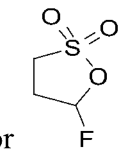 ;

and

the compound of formula III is:

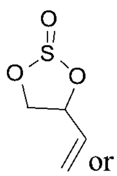 or 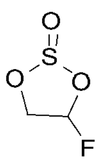 .

Therefore, the electrolyte of this application, by including the compound I and compound II or III of the foregoing structure, further enables the corresponding battery to have a high voltage and exhibit good cycling performance and storage performance at high temperature.

In some embodiments, the percentage of the compound of formula I in the electrolyte is a wt%, and the

percentage of the compound of formula II, the compound of formula III, or the mixture of the two, in the electrolyte is b wt%, where

$$0.05 \leq a/b \leq 4,$$

, preferably, $0.05 \leq a/b \leq 2$, further preferably, $0.5 \leq a/b \leq 1$.

Therefore, including the compound I and compound II or III with their respective percentages further enables the corresponding battery to have a high voltage and exhibit good cycling performance and storage performance at high temperature.

In some embodiments, the percentage of the compound of formula I in the electrolyte is a wt%, and the percentage of the compound of formula II, the compound of formula III, or the mixture of the two, in the electrolyte is b wt%, where

$$0.1 \leq a + b \leq 10,$$

preferably, $0.1 \leq a + b \leq 6$.

Therefore, including the compound I and compound II or III with their respective percentages further enables the corresponding battery to have a high voltage and exhibit good cycling performance and storage performance at high temperature.

In some embodiments, the percentage of the compound of formula II, the compound of formula III, or a mixture of the two, in the electrolyte is 0.06wt% to 8wt%, preferably, 1wt% to 8wt%.

Therefore, including the compound II or III with the percentage further enables the corresponding battery to have a high voltage and exhibit good cycling performance and storage performance at high temperature.

In some embodiments, the solvent is a non-protonic organic solvent, the non-protonic organic solvent being selected from one or more of fluorinated and non-fluorinated cyclic and chain organic carbonate, fluorinated and non-fluorinated ether, fluorinated and non-fluorinated cyclic ether, fluorinated and non-fluorinated carboxylic acid ester, and fluorinated and non-fluorinated chain sulfone or cyclic sulfone compounds, preferably, from one or more of fluorinated carbonate and fluorinated carboxylic acid ester.

Therefore, including the solvent of this type further enables the corresponding battery to have a high voltage and exhibit good cycling performance and storage performance at high temperature.

In some embodiments, the solvent may be selected from one or more of fluoroethylene carbonate (FEC), ethylene carbonate (EC), propylene carbonate (PC), ethyl methyl carbonate (EMC), diethyl carbonate (DEC), dimethyl carbonate (DMC), dipropyl carbonate (DPC), methyl propyl carbonate (MPC), ethyl propyl carbonate (EPC), butylene carbonate (BC), methyl butyrate (MB), ethyl butyrate (EB), 1,4-butyrolactone (GBL), sulfolane (SF), methyl sulfonyl methane (MSM), ethyl methyl sulfone (EMS), and diethyl sulfone (ESE).

In some embodiments, the lithium salt is selected from one or more of lithium hexafluorophosphate ($LiPF_6$), lithium tetrafluoroborate ($LiBF_4$), lithium perchlorate ($LiClO_4$), lithium hexafluoroarsenate ($LiAsF_6$), lithium bis(fluorosulfonyl)imide (LiFSI), lithium bistrifluoromethanesulfonimide (LiTFSI), lithium trifluoromethanesulfonate (LiTFS), lithium difluoro(oxalato)borate (LiDFOB), lithium dioxalate borate (LiBOB), lithium difluorophosphate ($LiPO_2F_2$), lithium difluoro(dioxalato)phosphate (LiDFOP), and lithium tetrafluoro oxalato phosphate (LiTFOP), and is optionally one or two of $LiPF_6$ and LiFSI.

Therefore, including the lithium salt of this type further enables the corresponding battery to have a high voltage and exhibit good cycling performance and storage performance at high temperature.

In some embodiments, in the electrolyte:

the solvent is 60wt% to 85wt%, preferably 80wt% to 88wt%,
the additive composition is 0.01wt% to 20wt%, preferably 0.3wt% to 15wt%; and
the lithium salt is 10wt% to 40wt%, preferably 10wt% to 15wt%; and
a total weight of the foregoing components is 100wt% and each wt% is based on a total weight of the electrolyte.

Therefore, including the components with their respective percentages further enables the corresponding battery to have a high voltage and exhibit good cycling performance and storage performance at high temperature.

In some embodiments, the additive composition may further include a negative electrode film-forming additive or a positive electrode film-forming additive or may include an additive capable of improving some performance of a battery, for example, an additive for improving overcharge performance of the battery, or an additive for improving high-temperature or low-temperature performance of the battery, such as lithium difluorophosphate ($LiPO_2F_2$) and lithium

fluorosulfate ($LiSO_3F$). The negative electrode film-forming additive is one or more of vinylene carbonate, fluoroethylene carbonate, or vinyl sulfate.

A second aspect of this application further provides a secondary battery characterized by including: the electrolyte according to the first aspect of this application.

Therefore, the battery has a high voltage and exhibits good cycling performance and storage performance at high temperature.

In addition, the following describes a secondary battery, a battery module, a battery pack, and an electric apparatus in this application with appropriate reference to the accompanying drawings.

Normally, the secondary battery includes a positive electrode plate, a negative electrode plate, an electrolyte, and a separator. In a charge and discharge process of the battery, active ions are intercalated and deintercalated between the positive electrode plate and the negative electrode plate. The electrolyte conducts ions between the positive electrode plate and the negative electrode plate. The separator is disposed between the positive electrode plate and the negative electrode plate to mainly prevent a short circuit between positive and negative electrodes and to allow the ions to pass through.

[Positive electrode plate]

The positive electrode plate includes a positive electrode current collector and a positive-electrode film layer disposed on at least one surface of the positive-electrode current collector, and the positive-electrode film layer includes the positive-electrode active material in the first aspect of this application.

For example, the positive electrode current collector includes two opposite surfaces in its thickness direction, and the positive electrode film layer is disposed on either or both of the two opposite surfaces of the positive electrode current collector.

In some embodiments, the positive electrode current collector may be a metal foil current collector or a composite current collector. For example, an aluminum foil may be used as the metal foil. The composite current collector may include a polymer material matrix and a metal layer formed on at least one surface of the polymer material matrix. The composite current collector may be formed by forming a metal material (aluminum, aluminum alloy, nickel, nickel alloy, titanium, titanium alloy, silver, silver alloy, or the like) on a polymer material matrix (for example, matrices of polypropylene (PP), polyethylene terephthalate (PET), polybutylene terephthalate (PBT), polystyrene (PS), and polyethylene (PE)).

In some embodiments, the positive electrode active material may be a well-known positive electrode active material used for batteries in the art. For example, the positive electrode active material may include at least one of the following materials: olivine-structured lithium-containing phosphate, lithium transition metal oxide, and respective modified compounds thereof. However, this application is not limited to such materials, and may alternatively use other conventional well-known materials that can be used as positive electrode active materials for batteries. One of these positive electrode active materials may be used alone, or two or more of them may be used in combination. Examples of the lithium transition metal oxide may include but are not limited to at least one of lithium cobalt oxide (for example, $LiCoO_2$), lithium nickel oxide (for example, $LiNiO_2$), lithium manganese oxide (for example, $LiMnO_2$ and $LiMn_2O_4$), lithium nickel cobalt oxide, lithium manganese cobalt oxide, lithium nickel manganese oxide, lithium nickel cobalt manganese oxide (for example, $LiNi_{1/3}Co_{1/3}Mn_{1/3}O_2$ ($NCM_{333}$ for short), $LiNi_{0.5}Co_{0.2}Mn_{0.3}O_2$ ($NCM_{523}$ for short), $LiNi_{0.5}Co_{0.25}Mn_{0.25}O_2$ ($NCM_{211}$ for short), $LiNi_{0.6}Co_{0.2}Mn_{0.2}O_2$ ($NCM_{622}$ for short), and $LiNi_{0.8}Co_{0.1}Mn_{0.1}O_2$ ($NCM_{811}$ for short)), lithium nickel cobalt aluminum oxide (for example, $LiNi_{0.85}Co_{0.15}Al_{0.05}O_2$), and modified compounds thereof. Examples of the olivine-structured lithium-containing phosphate may include but are not limited to at least one of lithium iron phosphate (for example, $LiFePO_4$ (LFP for short)), a composite material of lithium iron phosphate and carbon, lithium manganese phosphate (for example, $LiMnPO_4$), composite materials of lithium manganese phosphate and carbon, lithium manganese iron phosphate, and composite materials of lithium manganese iron phosphate and carbon.

In some embodiments, the positive electrode film layer may further optionally include a binder. For example, the binder may include at least one of polyvinylidene fluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, or fluorine-containing acrylic resin.

In some embodiments, the positive electrode film layer further optionally includes a conductive agent. For example, the conductive agent may include at least one of superconducting carbon, acetylene black, carbon black, Ketjen black, carbon dots, carbon nanotubes, graphene, and carbon nanofiber.

In some embodiments, the positive electrode plate may be prepared in the following manner: the foregoing constituents used for preparing the positive electrode plate, for example, the positive electrode active material, the conductive agent, the binder, and any other constituent, are dispersed in a solvent (for example, N-methylpyrrolidone) to form a positive electrode slurry; and the positive electrode slurry is applied onto the positive electrode current collector, followed by processes such as drying and cold pressing to obtain the positive electrode plate.

[Negative electrode plate]

The negative electrode plate includes a negative electrode current collector and a negative electrode film layer disposed on at least one surface of the negative electrode current collector, where the negative electrode film layer includes a negative electrode active material.

For example, the negative electrode current collector includes two opposite surfaces in its thickness direction, and the negative electrode film layer is disposed on either or both of the two opposite surfaces of the negative electrode current collector.

In some embodiments, the negative electrode current collector may be a metal foil current collector or a composite current collector. For example, for the metal foil, a copper foil may be used. The composite current collector may include a polymer material matrix and a metal layer formed on at least one surface of the polymer material matrix. The composite current collector may be formed by forming a metal material (copper, copper alloy, nickel, nickel alloy, titanium, titanium alloy, silver, silver alloy, or the like) on a polymer material matrix (for example, matrices of polypropylene (PP), polyethylene terephthalate (PET), polybutylene terephthalate (PBT), polystyrene (PS), and polyethylene (PE)).

In some embodiments, the negative electrode active material may be a well-known negative electrode active material used for batteries in the art. For example, the negative electrode active substance may include at least one of the following materials: artificial graphite, natural graphite, soft carbon, hard carbon, a phosphorus-based material, a tin-based material, lithium titanate, or the like. The silicon-based material may be selected from at least one of elemental silicon, silicon-oxygen compound, silicon-carbon composite, silicon-nitrogen composite, and silicon alloy. The tin-based material may be selected from at least one of elemental tin, tin-oxygen compound, and tin alloy. However, this application is not limited to these materials but may use other conventional materials that can be used as negative electrode active materials for batteries instead. One of these negative electrode active materials may be used alone, or two or more of them may be used in combination.

In some embodiments, the negative electrode film layer further optionally includes a binder. The binder may be selected from at least one of styrene-butadiene rubber (SBR), polyacrylic acid (PAA), polyacrylic acid sodium (PAAS), polyacrylamide (PAM), polyvinyl alcohol (PVA), sodium alginate (SA), polymethacrylic acid (PMAA), and carboxymethyl chitosan (CMCS).

In some embodiments, the negative-electrode film layer further optionally includes a conductive agent. The conductive agent may be selected from at least one of superconducting carbon, acetylene black, carbon black, Ketjen black, carbon dots, carbon nanotubes, graphene, and carbon nanofiber.

In some embodiments, the negative electrode film layer may further optionally include other promoters such as a thickener (for example, sodium carboxymethyl cellulose (CMC-Na)).

In some embodiments, the negative electrode plate may be prepared in the following manner: the constituents used for preparing the negative electrode plate, for example, the negative electrode active material, the conductive agent, the binder, and any other constituent, are dispersed in a solvent (for example, deionized water) to form a negative electrode slurry; and the negative electrode slurry is applied onto the negative electrode current collector, followed by processes such as drying and cold pressing to obtain the negative electrode plate.

[Electrolyte]

The electrolyte conducts ions between the positive electrode plate and the negative electrode plate.

In some embodiments, the electrolyte is the electrolyte according to the first aspect of this application.

[Separator]

In some embodiments, the secondary battery further includes a separator. The separator is not limited to any particular type in this application and may be any well-known porous separator with good chemical stability and mechanical stability.

In some embodiments, a material of the separator may be selected from at least one of glass fiber, non-woven fabric, polyethylene, polypropylene, and polyvinylidene fluoride. The separator may be a single-layer film or a multi-layer composite film and is not particularly limited. When the separator is a multi-layer composite film, all layers may be made of same or different materials, which is not particularly limited.

In some embodiments, the positive electrode plate, the negative electrode plate, and the separator may be made into an electrode assembly through winding or lamination.

In some embodiments, the secondary battery may include an outer package. The outer package is used for packaging the electrode assembly and the electrolyte.

In some embodiments, the outer package of the secondary battery may be a hard shell, for example, a hard plastic shell, an aluminum shell, or a steel shell. The outer package of the secondary battery may alternatively be a soft

package, for example, a soft pouch. A material of the soft pack may be plastic. As the plastic, polypropylene, polybutylene terephthalate, polybutylene succinate, and the like may be listed.

This application does not impose any special limitations on a shape of the secondary battery, and the secondary battery may be cylindrical, rectangular, or of any other shapes. For example, FIG. 1 shows a secondary battery 5 of a rectangular structure as an example.

In some embodiments, referring to FIG. 2, the outer package may include a housing 51 and a cover plate 53. The housing 51 may include a base plate and a side plate connected to the base plate, where the base plate and the side plate enclose an accommodating cavity. The housing 51 has an opening communicating with the accommodating cavity, and the cover plate 53 can cover the opening to close the accommodating cavity. The positive electrode plate, the negative electrode plate, and the separator may be made into an electrode assembly 52 through winding or lamination. The electrode assembly 52 is packaged in the accommodating cavity. Electrolyte infiltrates into the electrode assembly 52. There may be one or more electrode assemblies 52 in the secondary battery 5, and persons skilled in the art may make choices according to actual requirements.

In some embodiments, the secondary battery may be assembled into a battery module, and the battery module may include one or more secondary batteries. The specific quantity may be chosen by persons skilled in the art according to the use and capacity of the battery module.

FIG. 3 shows a battery module 4 as an example. With reference to FIG. 3, in the battery module 4, a plurality of secondary batteries 5 may be sequentially arranged in a length direction of the battery module 4. Certainly, the batteries may alternatively be arranged in any other manners. Further, the plurality of secondary batteries 5 may be fastened through fasteners.

Optionally, the battery module 4 may further include a housing with accommodating space, and the plurality of secondary batteries 5 are accommodated in the accommodating space.

In some embodiments, the battery module may be further assembled into a battery pack, and the battery pack may include one or more battery modules. The specific quantity may be chosen by persons skilled in the art according to the use and capacity of the battery pack.

FIG. 4 and FIG. 5 show a battery pack 1 as an example. Referring to FIG. 4 and FIG. 5, the battery pack 1 may include a battery box and a plurality of battery modules 4 arranged in the battery box. The battery box includes an upper box body 2 and a lower box body 3. The upper box body 2 can cover the lower box body 3 to form an enclosed space for accommodating the battery module 4. The plurality of battery modules 4 may be arranged in the battery box in any manner.

In addition, this application further provides an electric apparatus. The electric apparatus includes at least one of the secondary battery, the battery module, or the battery pack provided in this application. The secondary battery, the battery module, or the battery pack may be used as a power source for the electric apparatus or an energy storage unit of the electric apparatus. The electric apparatus may include a mobile device (for example, a mobile phone or a notebook computer), an electric vehicle (for example, a battery electric vehicle, a hybrid electric vehicle, a plug-in hybrid electric vehicle, an electric bicycle, an electric scooter, an electric golf vehicle, or an electric truck), an electric train, a ship, a satellite system, an energy storage system, or the like, but is not limited thereto.

The secondary battery, the battery module, or the battery pack may be selected for the electric apparatus based on requirements for using the electric apparatus.

FIG. 6 shows an electric apparatus as an example. The electric apparatus is a battery electric vehicle, a hybrid electric vehicle, a plug-in hybrid electric vehicle, or the like. To satisfy a requirement of the electric apparatus for high power and high energy density of the secondary battery, a battery pack or a battery module may be used.

In another example, the apparatus may be a mobile phone, a tablet computer, a notebook computer, or the like. The apparatus usually requires to be light and thin, and a secondary battery may be used as a power source.

## Examples

To describe the technical problems solved by this application, technical solutions, and beneficial effects of this application more clearly, the following further describes this application in detail with reference to the embodiments and accompanying drawings. Apparently, the described embodiments are merely some but not all of the embodiments of this application. The following description of at least one exemplary embodiment is merely illustrative and definitely is not construed as any limitation on this application or on use of this application. All other embodiments obtained by a person of ordinary skill in the art based on the embodiments of this application without creative efforts shall fall within the protection scope of this application.

Examples whose technical solutions or conditions are not specified are made in accordance with technical solutions or conditions described in literature in the field or made in accordance with product instructions. The reagents or instruments used are all conventional products that are commercially available if no manufacturer is indicated.

### Example 1

(1) Preparation of positive electrode plate

Single crystal particles, as a positive electrode active material, $LiNi_{0.5}Mn_{0.3}Co_{0.2}O_2$ with $D_v50$ of 3.8 μm, an acetylene black conductive agent, and a polyvinylidene fluoride (PVDF) binder were fully stirred and uniformly mixed in an N-methylpyrrolidone (NMP) solvent at a weight ratio of 96:2:2 to obtain a positive electrode slurry, and then the positive electrode slurry was applied onto the positive electrode current collector, followed by drying, cold pressing, and cutting to obtain a positive electrode plate.

(2) Preparation of negative-electrode plate

A negative electrode active material graphite (with BET of 1.2 $m^3/g$, and $D_{v50}$ of 18 μm), a conductive agent acetylene black, a binder styrene-butadiene rubber (SBR), and a thickener sodium carboxymethyl cellulose (CMC) were dissolved in a solvent deionized water at a weight ratio of 95:2:2:1 and were uniformly mixed with the solvent deionized water to obtain a negative electrode slurry. The negative electrode slurry was uniformly applied onto a negative electrode current collector copper foil, followed by drying to obtain a negative electrode film, and then followed by cold pressing and cutting to obtain a negative electrode plate.

(3) Separator

A commercially available polypropylene film was used as a separator.

(4) Preparation of electrolyte

In an argon atmosphere glove box ($H_2O$ < 0.1ppm, $O_2$ < 0.1ppm), the organic solvents EC/EMC were mixed uniformly at a volume ratio of 3:7. Then, 1M LiPF6 lithium salt was dissolved in the organic solvent. Additionally, 0.5wt% tetravinylsilicon and 1wt% 2-fluoro-1,3-propane sultone, calculated based on the weight of the electrolyte were added. The mixture was stirred uniform to obtain the corresponding electrolyte.

(5) Preparation of battery

The positive electrode plate, the separator, and the negative electrode plate were sequentially stacked so that the separator was located between the positive electrode plate and the negative electrode plate for separation. Then, the resulting stack was wound to obtain an electrode assembly. The electrode assembly was placed into a battery housing and dried. The electrolyte was injected, and processes such as formation and standing were performed to obtain a lithium-ion battery.
Preparation methods of the secondary batteries in examples 2 to 19 and comparative examples 1 to 4 were similar to the preparation method of the secondary battery in example 1, but compositions and product parameters of the additive, the solvent, and the positive electrode active material in the electrolyte were adjusted. For details, see table 1.

Table 1: Relevant parameters and compositions of various examples and comparative examples of secondary batteries

| Example number | Compound I | Compound II | | | Compound III | | Other additives | | | | Solvent | Positive electrode active material |
|---|---|---|---|---|---|---|---|---|---|---|---|---|
| | 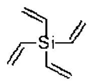 | 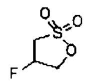 | 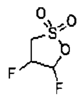 | 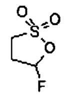 | 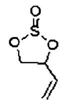 | 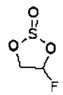 | 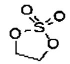 | 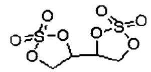 | LiPO2F2 | LiSO3F | | |
| Comparative example 1 | No additive | | | | | | | | | | EC/EMC=3/7 | Single crystal/3.8 μm |
| Comparative example 2 | 2 | 0 | 0 | 0 | 0 | 0 | 0 | 0 | 0 | 0 | EC/EMC=3/7 | Single crystal/3.8 μm |
| Comparative example 3 | 0 | 1 | 0 | 0 | 0 | 0 | 0 | 0 | 0 | 0 | EC/EMC=3/7 | Single crystal/3.8 μm |
| Comparative example 4 | 0 | 0 | 0 | 0 | 1 | 0 | 0 | 0 | 0 | 0 | EC/EMC=3/7 | Single crystal/3.8 μm |
| Comparative example 5 | 0.5 | Compound II is 1,3-propane sultone with a concentration of 1 | | | 0 | 0 | 0 | 0 | 0 | 0 | EC/EMC=3/7 | Single crystal/3.8 μm |
| Comparative example 6 | 0.5 | 0 | 0 | 0 | Compound II is ethyl sulfite with a concentration of 1 | | | 0 | 0 | 0 | EC/EMC=3/7 | Single crystal/3.8 μm |
| 1 | 0.5 | 1 | 0 | 0 | 0 | 0 | 0 | 0 | 0 | 0 | EC/EMC=3/7 | Single crystal/3.8 μm |
| 2 | 0.5 | 0 | 1 | 0 | 0 | 0 | 0 | 0 | 0 | 0 | EC/EMC=3/7 | Single crystal/3.8 μm |
| 3 | 0.5 | 0 | 0 | 1 | 0 | 0 | 0 | 0 | 0 | 0 | EC/EMC=3/7 | Single crystal/3.8 μm |
| 4 | 0.5 | 0 | 0 | 0 | 1 | 0 | 0 | 0 | 0 | 0 | EC/EMC=3/7 | Single crystal/3.8 μm |
| 5 | 0.5 | 0 | 0 | 0 | 0 | 1 | 0 | 0 | 0 | 0 | EC/EMC=3/7 | Single crystal/3.8 μm |
| 6 | 0.5 | 0.5 | 0 | 0 | 0.5 | 0 | 0 | 0 | 0 | 0 | EC/EMC=3/7 | Single crystal/3.8 μm |
| 7 | 0.2 | 0 | 0 | 2 | 0 | 2 | 0 | 0 | 0 | 0 | EC/EMC=3/7 | Single crystal/3.8 μm |

(continued)

| Example number | Compound I | Compound II | | | Compound III | | Other additives | | | | Solvent | Positive electrode active material |
|---|---|---|---|---|---|---|---|---|---|---|---|---|
| | 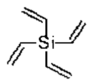 | 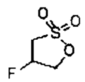 | 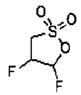 | 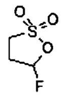 | 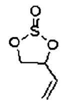 | 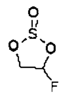 | 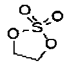 | 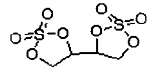 | LiPO2F2 | LiSO3F | | |
| 8 | 1 | 0.5 | 0 | 0 | 0.5 | 0 | 0 | 0 | 0 | 0 | EC/EMC=3/7 | Single crystal/3.8 μm |
| 9 | 2 | 0.5 | 0 | 0 | 0.5 | 0 | 0 | 0 | 0 | 0 | EC/EMC=3/7 | Single crystal/3.8 μm |
| 10 | 4 | 1 | 0 | 0 | 0 | 0 | 0 | 0 | 0 | 0 | EC/EMC=3/7 | Single crystal/3.8 μm |
| 11 | 5 | 1 | 0 | 0 | 0 | 0 | 0 | 0 | 0 | 0 | EC/EMC=3/7 | Single crystal/3.8 μm |
| 12 | 0.033 | 0 | 0.033 | 0 | 0.034 | 0 | 0 | 0 | 0 | 0 | EC/EMC=3/7 | Single crystal/3.8 μm |
| 13 | 3.3 | 0 | 3.3 | 0 | 3.4 | 0 | 0 | 0 | 0 | 0 | EC/EMC=3/7 | Single crystal/3.8 μm |
| 14 | 4 | 0 | 4 | 0 | 4 | 0 | 0 | 0 | 0 | 0 | EC/EMC=3/7 | Single crystal/3.8 μm |
| 15 | 0.5 | 0 | 0.5 | 0 | 0.5 | 0 | 0.5 | 0 | 1 | 0 | EC/EMC=3/7 | Single crystal/3.8 μm |
| 16 | 0.5 | 0 | 0.5 | 0 | 0.5 | 0 | 0.5 | 0.5 | 1 | 1 | EC/EMC=3/7 | Single crystal/3.8 μm |
| 17 | 0.5 | 0 | 0.5 | 0 | 0.5 | 0 | 0.5 | 0.5 | 1 | 1 | EC/FEMC=3/7 | Single crystal/3.8 μm |
| 18 | 0.5 | 0 | 0.5 | 0 | 0.5 | 0 | 0.5 | 0.5 | 1 | 1 | EC/FEMC/fluorobenzene =2/7/1 | Single crystal/3.8 μm |
| 19 | 0.5 | 0.5 | 0 | 0 | 0.5 | 0 | 0 | 0 | 0 | 0 | EC/EMC=3/7 | Polycrystalline/3.8 μm |
| 20 | 0.5 | 0.5 | 0 | 0 | 0.5 | 0 | 0 | 0 | 0 | 0 | EC/EMC=3/7 | Single crystal/1 μm |

(continued)

| Example number | Compound I | Compound II | | | Compound III | | Other additives | | | | Solvent | Positive electrode active material |
|---|---|---|---|---|---|---|---|---|---|---|---|---|
| | 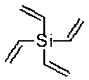 | 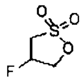 | 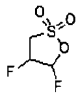 | 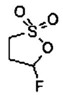 | 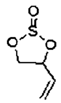 | 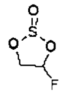 | 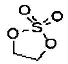 | 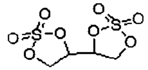 | LiPO2F2 | LiSO3F | | |
| 21 | 0.5 | 0.5 | 0 | 0 | 0.5 | 0 | 0 | 0 | 0 | 0 | EC/EMC=3/7 | Single crystal/15 μm |

**2. Battery performance testing**

(1) Cycling performance testing on the lithium-ion battery at 25°C

At 25°C, the lithium-ion battery was charged to 4.45V at a constant current of 0.5C, then charged to a current less than 0.05C at a constant voltage of 4.45V, and then discharged to 2.8V at a constant current of 0.5C, to obtain the first-cycle discharge capacity C0, which was recorded as the first cycle. Charging and discharging were performed as such repeatedly, to calculate the number of cycles when the discharge capacity of the lithium-ion battery reached 80% of C0.

(2) Cycling performance testing on the lithium-ion battery at 45°C

At 45°C, the lithium-ion battery was charged to 4.45V at a constant current of 1C, then charged to a current less than 0.05C at a constant voltage of 4.45V, and then discharged to 2.8V at a constant current of 1C, to obtain the first-cycle discharge capacity C0, which was recorded as the first cycle. Charging and discharging were performed as such repeatedly, to calculate the number of cycles when the discharge capacity of lithium-ion battery reached 80% of C0.

(3) Storage performance testing of the lithium-ion battery at 60°C

At 25°C, the lithium-ion battery was charged to 4.45V at a constant current of 0.5C and then charged to a current less than 0.05C at a constant voltage of 4.45V. Then, the cell was stored at 60°C. Every 10 days, the cell was taken out, fully charged, and then returned to storage. This process was repeated for a total of 100 days, and the corresponding remaining reversible capacity was recorded.

**3. Test results of examples and comparative examples**

Batteries in the examples and comparative examples were prepared according to the foregoing methods, and performance parameters were measured. Results were shown in the table 2.

**Table 2 Battery performance of examples and comparative examples**

| Example number | Cycling life/cycles at 25°C | Cycling life/cycles at 45°C | Capacity retention rate after storage at 60°C for 100 days |
|---|---|---|---|
| Comparative example 1 | 303 | 102 | 12% |
| Comparative example 2 | 553 | 223 | 31% |
| Comparative example 3 | 501 | 207 | 27% |
| Comparative example 4 | 527 | 216 | 28% |
| Comparative example 5 | 688 | 278 | 39% |
| Comparative example 6 | 504 | 202 | 24% |
| 1 | 1374 | 704 | 92.80% |
| 2 | 1369 | 706 | 92.50% |
| 3 | 1395 | 717 | 92.70% |
| 4 | 1434 | 728 | 93.50% |
| 5 | 1389 | 718 | 93.60% |
| 6 | 1427 | 722 | 93.00% |
| 7 | 1354 | 714 | 92.70% |
| 8 | 1437 | 738 | 93.52% |
| 9 | 1368 | 723 | 92.89% |
| 10 | 1289 | 677 | 91.60% |
| 11 | 1043 | 563 | 86.80% |
| 12 | 1272 | 665 | 89.50% |
| 13 | 1206 | 654 | 89.00% |

(continued)

| Example number | Cycling life/cycles at 25°C | Cycling life/cycles at 45°C | Capacity retention rate after storage at 60°C for 100 days |
|---|---|---|---|
| 14 | 1008 | 541 | 86.80% |
| 15 | 1765 | 922 | 94.80% |
| 16 | 1824 | 1009 | 95.20% |
| 17 | 1890 | 1022 | 96.20% |
| 18 | 1910 | 1052 | 96.50% |
| 19 | 932 | 379 | 68.00% |
| 20 | 775 | 318 | 54.00% |
| 21 | 828 | 341 | 78.00% |

It can be seen from table 1 and table 2 that the electrolyte in the battery of the present invention, by including the compound of formula I and the compound of formula II and III, improves the high-temperature cycling life and high-temperature storage performance of the battery.

Further embodiments of the present invention are provided in the following. It should be noted that the numbering used in the following section does not necessarily need to comply with the numbering used in the previous sections.

Embodiment 1. An electrolyte for lithium-ion secondary battery, comprising a solvent, an additive composition, and a lithium salt, characterized in that the additive composition comprises a compound of formula I and at least one of compounds of formula II and formula III:

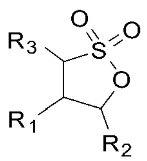

formula II

wherein $R_1$, $R_2$, and $R_3$ are each independently H or F, and at least one of $R_1$, $R_2$, and $R_3$ is F;

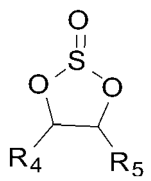

formula III

wherein $R_4$ and $R_5$ are each independently hydrogen, vinyl, allyl, oxyvinyl, or oxyallyl, and at least one of $R_4$ and $R_5$ is not H; and

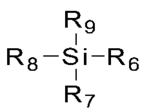

formula I

wherein $R_6$, $R_7$, $R_8$, and $R_9$ are each independently selected from one or more of allyl, vinyl, or allyl butyl.

Embodiment 2. The electrolyte according to embodiment 1, characterized in that the compound of formula I is:

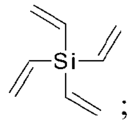 ;

the compound of formula II is:

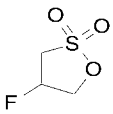 , 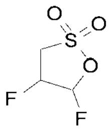 , or 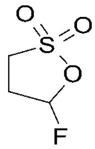 ;

and

the compound of formula III is:

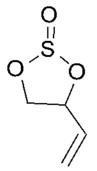 or 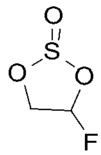 .

Embodiment 3. The electrolyte according to embodiment 1 or 2, characterized in that percentage of the compound of formula I in the electrolyte is a wt%, and percentage of the compound of formula II, the compound of formula III, or a mixture of the two, in the electrolyte is b wt%, wherein

$$0.05 \leq a/b \leq 4.$$

Embodiment 4. The electrolyte according to any one of embodiments 1 to 3, characterized in that percentage of the compound of formula I in the electrolyte is a wt%, and percentage of the compound of formula II, the compound of formula III, or a mixture of the two, in the electrolyte is b wt%, wherein

$$0.1 \leq a+b \leq 10.$$

Embodiment 5. The electrolyte according to any one of embodiments 1 to 4, characterized in that percentage of the compound of formula II, the compound of formula III, or a mixture of the two, in the electrolyte is 0.06wt% to 8wt%.

Embodiment 6. The electrolyte according to any one of embodiments 1 to 5, characterized in that the solvent is a non-protonic organic solvent, the non-protonic organic solvent being selected from one or more of fluorinated and non-fluorinated cyclic and chain organic carbonate, fluorinated and non-fluorinated ether, fluorinated and non-fluorinated cyclic ether, fluorinated and non-fluorinated carboxylic acid ester, and fluorinated and non-fluorinated chain sulfone or cyclic sulfone compounds.

Embodiment 7. The electrolyte according to any one of embodiments 1 to 6, characterized in that the lithium salt is selected from one or more of lithium hexafluorophosphate, lithium tetrafluoroborate, lithium perchlorate, lithium hexafluoroarsenate, lithium bis(fluorosulfonyl)imide, lithium bis(trifluoromethanesulfonyl)imide, lithium trifluoromethanesulfonate, lithium difluoroxalate borate, lithium dioxalate borate, lithium difluorophosphate, lithium difluoro(dioxalato)phosphate, and lithium tetrafluoro oxalato phosphate, and optionally is selected from one or two of lithium hexafluorophosphate or lithium bis(fluorosulfonyl)imide.

Embodiment 8. The electrolyte according to any one of embodiments 1 to 7, characterized in that in the electrolyte,

the solvent is 60wt% to 85wt%.
the additive composition is 0.01wt% to 20wt%, and
the lithium salt is 10wt% to 40wt%; and
a total weight of the foregoing components is 100wt% and each wt% is based on a total weight of the electrolyte.

Embodiment 9. A secondary battery, characterized by comprising:
the electrolyte according to any one of embodiments 1 to 8.

Embodiment 10. A battery module, characterized by comprising the secondary battery according to embodiment 9.

Embodiment 11. A battery pack, characterized by comprising the battery module according to embodiment 10.

Embodiment 12. An electric apparatus, characterized by comprising at least one of the secondary battery according to embodiment 9, the battery module according to embodiment 10, or the battery pack according to embodiment 11.

It should be noted that this application is not limited to the foregoing embodiments. The foregoing embodiments are merely examples, and embodiments having substantially the same constructions and the same effects as the technical idea within the scope of the technical solutions of this application are all included in the technical scope of this application. In addition, without departing from the essence of this application, various modifications made to the embodiments that can be conceived by persons skilled in the art, and other manners constructed by combining some of the constituent elements in the embodiments are also included in the scope of this application.

## Claims

1. An electrolyte for lithium-ion secondary battery, comprising a solvent, an additive composition, and a lithium salt, **characterized in that** the additive composition comprises a compound of formula I

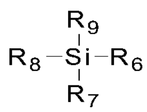

formula I

wherein $R_6$, $R_7$, $R_8$, and $R_9$ are each independently selected from one or more of allyl, vinyl, or allyl butyl; and a compound of formula III:

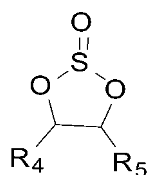

formula III

wherein $R_4$ and $R_5$ are each independently hydrogen, vinyl, allyl, oxyvinyl, or oxyallyl, and at least one of $R_4$ and $R_5$ is not H.

2. The electrolyte according to claim 1, wherein the additive composition further comprises a compound of formula II

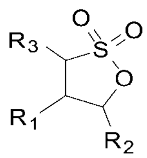

formula II

wherein $R_1$, $R_2$, and $R_3$ are each independently H or F, and at least one of $R_1$, $R_2$, and $R_3$ is F.

3. An electrolyte for lithium-ion secondary battery, comprising a solvent, an additive composition, and a lithium salt, **characterized in that** the additive composition comprises a compound of formula I:, :

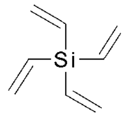

formula I;

and at least one of compounds of formula II and formula III:

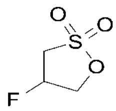,
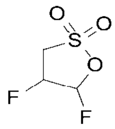, or
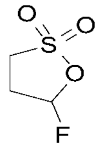

formula II,

and

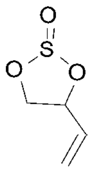
or
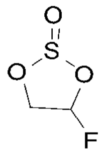

formula III.

**4.** The electrolyte according to any of claims 1 to 3, **characterized in that** percentage of the compound of formula I in the electrolyte is a wt%, and percentage of the compound of formula III, or a mixture of the compound of formula II and formula III, in the electrolyte is b wt%, wherein

$$0.05 \leq a/b \leq 4.$$

**5.** The electrolyte according to any one of claims 1 to 4, **characterized in that** percentage of the compound of formula I in the electrolyte is a wt%, and percentage of the compound of formula III, or a mixture of the compound of formula II and formula III, in the electrolyte is b wt%, wherein

$$0.1 \leq a+b \leq 10.$$

**6.** The electrolyte according to any one of claims 1 to 5, **characterized in that** percentage of the compound of formula III, or a mixture of the compound of formula II and formula III, in the electrolyte is 0.06wt% to 8wt%.

**7.** The electrolyte according to any one of claims 1 to 6, **characterized in that** the solvent is a non-protonic organic solvent, the non-protonic organic solvent being selected from one or more of fluorinated and non-fluorinated cyclic and chain organic carbonate, fluorinated and non-fluorinated ether, fluorinated and non-fluorinated cyclic ether, fluorinated and non-fluorinated carboxylic acid ester, and fluorinated and non-fluorinated chain sulfone or cyclic sulfone compounds.

**8.** The electrolyte according to any one of claims 1 to 7, **characterized in that** the lithium salt is selected from one or more of lithium hexafluorophosphate, lithium tetrafluoroborate, lithium perchlorate, lithium hexafluoroarsenate, lithium bis(fluorosulfonyl)imide, lithium bis(trifluoromethanesulfonyl)imide, lithium trifluoromethanesulfonate, lithium difluoroxalate borate, lithium dioxalate borate, lithium difluorophosphate, lithium difluoro(dioxalato)phosphate, and lithium tetrafluoro oxalato phosphate, and optionally is selected from one or two of lithium hexafluorophosphate or lithium bis(fluorosulfonyl)imide.

**9.** The electrolyte according to any one of claims 1 to 8, **characterized in that** in the electrolyte,

the solvent is 60wt% to 85wt%.
the additive composition is 0.01wt% to 20wt%, and
the lithium salt is 10wt% to 15wt%; and
a total weight of the foregoing components is 100wt% and each wt% is based on a total weight of the electrolyte.

**10.** A secondary battery (5), **characterized by** comprising:
the electrolyte according to any one of claims 1 to 9.

**11.** A battery module (4), **characterized by** comprising the secondary battery (5) according to claim 10.

**12.** A battery pack (1), **characterized by** comprising the battery module (4)according to claim 11.

**13.** An electric apparatus, **characterized by** comprising at least one of the secondary battery (5) according to claim 10, the battery module (4) according to claim 10, or the battery pack (1) according to claim 12.

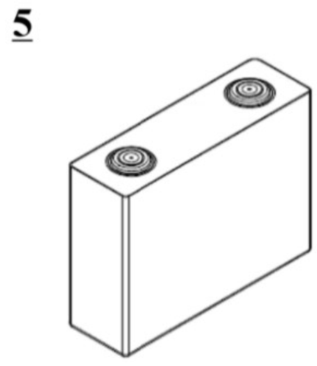


FIG. 1

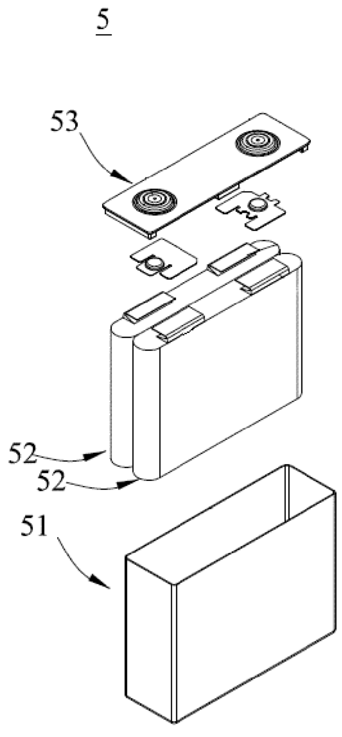


FIG. 2

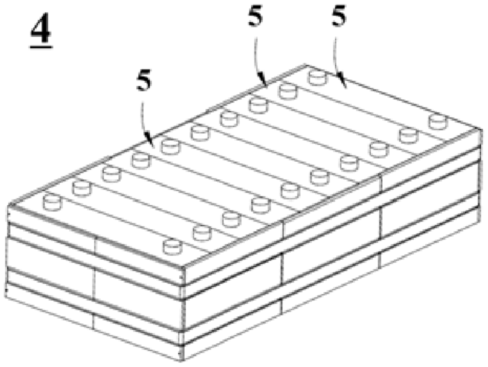


FIG. 3

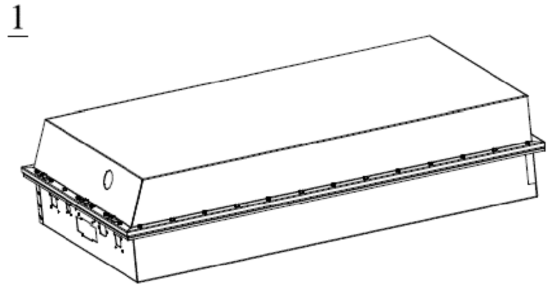


FIG. 4

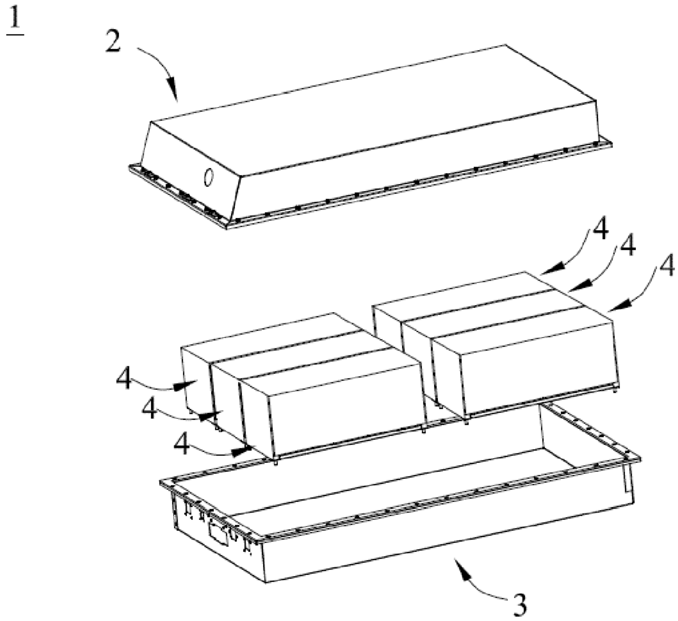


FIG. 5

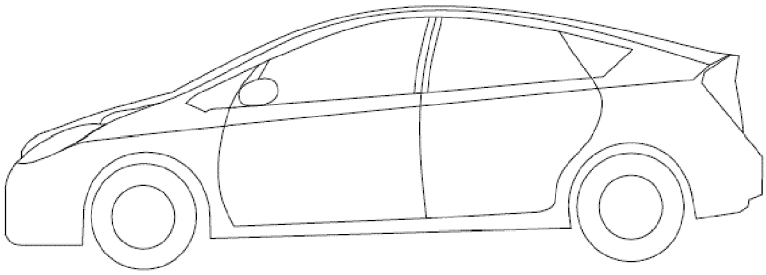

FIG. 6